# EUROPEAN PATENT SPECIFICATION

(11) **EP 1 284 276 B1**
(45) Date of publication and mention of the grant of the patent: **17.03.2010**
(21) Application number: 02015695.6
(22) Date of filing: 18.07.2002
(51) Int. Cl.: C08G 61/12, C07D 233/46

(54) **Conjugated copolymers of dithienothiophene with vinylene or acetylene**
Konjugierte Copolymere aus Dithienothiophen und Vinylen oder Acetylen
Copolymères conjugués de dithienothiophène et vinylène ou acetylène

(30) Priority: 17.08.2001 EP 01118894
(43) Date of publication of application: 19.02.2003
(73) Proprietor: Merck Patent GmbH, 64293 Darmstadt (DE)
(72) Inventor: Giles, Mark, Southampton, SO15 2LE (GB); Farrand, Louise Diane, Blandform Forum, Dorset, DT11 9ED (GB); Heeney, Martin, Southampton, SO14 6 TQ (GB); Shkunov, Maxim, Southampton, SO 16 6SX (GB); Sparrowe, David, Bournemouth, Dorset, BH6 5EJ (GB); Tierney, Steven, Southampton, SO15 7QW (GB); Thompson, Marcus, Fordingbridge, Hampshire, SP6 1RR (GB); McCulloch, Iain, Kings Somborne, Hampshire, SO20 6PE (GB)

(56) References cited:
- WO-A-99/12989
- KOSSMEHL G ET AL: "Über Polyarylenalkenylene und Polyheteroarylenalkenylene" MAKROMOLEKULARE CHEMIE, MACROMOLECULAR CHEMISTRY AND PHYSICS, HUTHIG UND WEPF VERLAG, BASEL, CH, vol. 183, no. 11, 1 November 1982 (1982-11-01), pages 2771-2786, XP002085568 ISSN: 0025-116X
- KIM OH-KIL ET AL: "Nonlinear optical chromophores containing dithienothiophene as a new type of electron relay" JOURNAL OF MATERIALS CHEMISTRY, THE ROYAL SOCIETY OF CHEMISTRY, CAMBRIDGE, GB, vol. 9, 1999, pages 2227-2232, XP002188214 ISSN: 0959-9428

## Description

### Field of Invention

The invention relates to new conjugated copolymers of dithienothiophene with vinylene or acetylene (ethinylene). The invention further relates to methods of their preparation, to their use as semiconductors or charge transport materials in optical, electrooptical or electronic devices including field effect transistors, electroluminescent, photovoltaic and sensor devices. The invention further relates to field effect transistors and semiconducting components comprising the new polymers.

### Background and Prior Art

Organic materials have recently shown promise as the active layer in organic based thin film transistors and organic field effect transistors [see reference 1]. Such devices have potential applications in smart cards, security tags and the switching element in flat panel displays. Organic materials are envisaged to have substantial cost advantages over their silicon analogues if they can be deposited from solution, as this enables a fast, large-area fabrication route.

The performance of the device is principally based upon the charge carrier mobility of the semiconducting material and the current on/off ratio, so the ideal semiconductor should have a low conductivity in the off state, combined with a high charge carrier mobility (> 1 x 10⁻³ cm² V⁻¹ s⁻¹). In addition, it is important that the semiconducting material is relatively stable to oxidation i.e. it has a high ionisation potential, as oxidation leads to reduced device performance.

Compounds known in prior art which have been shown to be effective *p*-type semiconductors for organic FETs are dithienothiophene (DTT) **(1)** and its fused dimer α,α'-bis(dithieno[3,2-b:2',3'-d]thiophene (BDT) (**2**) having the structures shown below [see reference 2-4].

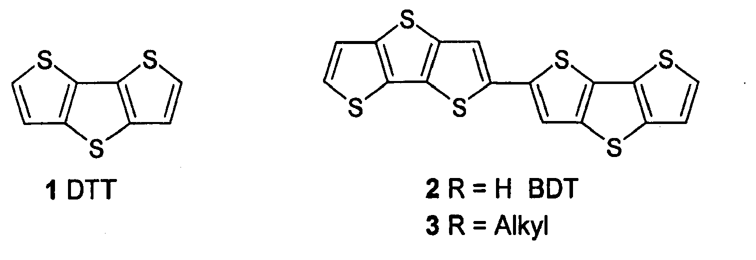

In particular BDT, which has been extensively studied, has been shown to be an effective p-type semiconductor for organic FETs with a very high charge carrier mobility between 1 x 10⁻³ and 5 x 10⁻² cm² V⁻¹ s⁻¹ and very high current on/off ratios (up to 10⁸). BDT also has been found in the solid state to have a completely coplanar formation, and to be more planar than oligomers of thiophene.

However, BDT has a high melting point and is very insoluble, therefore, if used as the active layer in an organic thin film transistor, it cannot be readily solution processed. As a result, for applications like FETs, prior art materials like BDT are usually deposited as a thin film by vacuum deposition, which is an expensive processing technique that is unsuitable for the fabrication of large-area films. To improve the solubility of BDT, several substituted derivatives have so far been synthesized (**3**), [see reference 4] but these have still required vacuum processing when used in thin film transistors.

It is the aim of the present invention to provide new materials for use as semiconductors or charge transport materials, which are easy to synthesize, have high charge mobility and are easily processible to form thin and large-area films for use in semiconductor devices. Other aims of the invention are immediately evident to those skilled in the art from the following description.

The inventors have found that these aims can be achieved by providing new vinylene or acetylene copolymers of dithienothiophene (DTT).

Polymers containing DTT have been previously synthesised. DTT can be polymerised electrochemically but an insoluble material containing many structural defects is produced [see reference 5,6]. Similarly copolymers with dithienopyrrole have also been made (**4**) [see reference 7]. DTT has also been incorporated into vinylidene polymers either via Knoevenagel (**5**) [see reference 8] or Wittig reactions (**6**) [see reference 9]. The latter gave insoluble polymers, whereas the former produced polymers that were soluble but had only moderate photovoltaic or photoconductive behaviour.

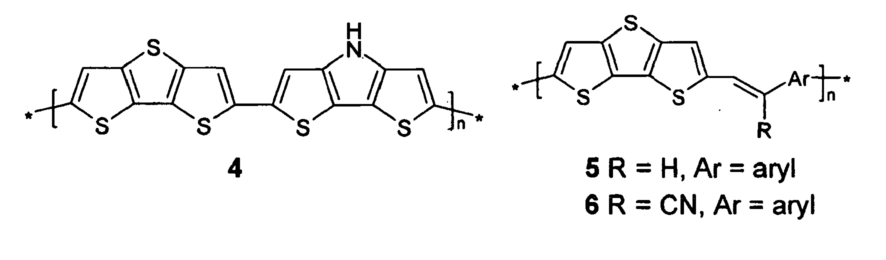

DTT dimers and homo polymers of DTT and copolymers of DTT and thiophenes are reported in the international patent application WO 99/12989 [reference 10]. However, no characterisation or details of the synthetic route of the polymers are disclosed.

Kossmehl et al., Makromol. Chem. 1982, 183(11), 2771-2786 discloses polymers built from repeating units containing a dithienothiophene group and a divinylarylene group. Kim et al., J. Mater. Chem. 1999, 9, 2227-2232 discloses nonlinear optical chromophores containing a dithienothiophene group, a donor group and an acceptor group. However, these documents do not disclose or suggest polymers as claimed in the present invention.

### Summary of the Invention

The invention relates to new conjugated copolymers as claimed in claim 1.

The invention further relates to the use of polymers according to the invention as semiconductors or charge transport materials, in particular in optical, electrooptical or electronic devices, like for example in field effect transistors (FET) as components of integrated circuitry, as thin film transistors in flat panel display applications or for Radio Frequency Identification (RFID) tags, or in semiconducting components for organic light emitting diode (OLED) applications such as electroluminescent displays or backlights of e.g. liquid crystal displays, for photovoltaic or sensor devices, as electrode materials in batteries, as photoconductors and for electrophotographic applications like electrophotographic recording.

The invention further relates to a field effect transistor, for example as a component of integrated circuitry, as a thin film transistor in flat panel display applications, or in a Radio Frequency Identification (RFID) tag, comprising one or more poly(dithienothiophene vinylenes) according to the invention.

The invention further relates to a semiconducting component, for example in OLED applications like electroluminescent displays or backlights of e.g. liquid crystal displays, in photovoltaic or sensor devices, as electrode materials in batteries, as photoconductors and for electrophotographic applications, comprising one or more poly(dithienothiophene vinylenes) according to the invention.

The invention further relates to a security marking or device comprising an RFID or ID tag or a FET according to the invention.

### Detailed Description of the Invention

The polymers have identical or different recurring units of formula I 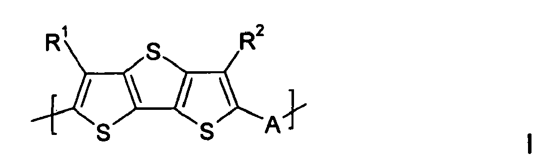 wherein
- R¹ and R²: are independently of each other H, halogen or straight chain, branched or cyclic alkyl with 1 to 20 C-atoms, which may be unsubstituted, mono- or poly-substituted by F, Cl, Br, I or CN, it being also possible for one or more non-adjacent CH₂ groups to be replaced, in each case independently from one another, by -O-, -S-, -NH-, -NR⁰-, -SiR⁰R⁰⁰- -CO-, -COO-, -OCO-, -OCO-O-, -S- CO-, -CO-S-, -CH=CH- or -C≡C- in such a manner that O and/or S atoms are not linked directly to one another, or optionally substituted aryl or heteroaryl,
- R⁰ and R⁰⁰: are independently of each other H or alkyl with 1 to 12 C-atoms,
- A: is -CX¹=CX²- or -C≡C-, and
- X¹ and X²: are independently of each other H, F, Cl or CN.

Particularly preferred are polymers of formulae I1 and I2 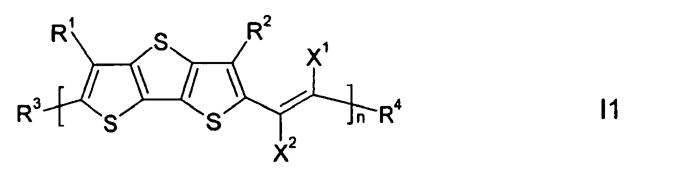 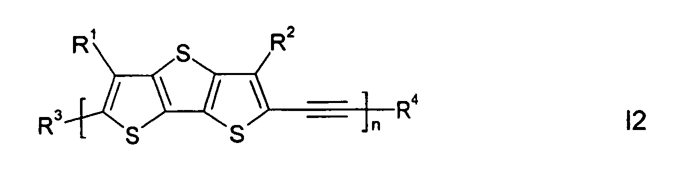 wherein R¹, R², R⁰, R⁰⁰, X¹ and X² have independently of each other one of the meanings of formula I,
- R³ and R⁴: are independently of each other H, halogen, Sn(R⁰)₃ or straight chain, branched or cyclic alkyl with 1 to 20 C- atoms, which may be unsubstituted, mono- or poly-substituted by F, Cl, Br, I, -CN and/ or -OH, it being also possible for one or more non-adjacent CH₂ groups to be replaced, in each case independently from one another, by -O-, -S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another, or optionally substituted aryl or heteroaryl,
- n: is an integer from 2 to 5000,
and wherein the recurring units may be identical or different.

Especially preferred are polymers of formula I, I1 and I2 having identical recurring units.

Further preferred are polymers of formula I, I1 and I2 wherein R¹ and R² are identical groups.

Especially preferred are polymers of formulae I, I1 and I2 having a degree of polymerisation (number of recurring units) from 10 to 5000, very preferably from 100 to 1000.

Further preferred are polymers of formulae I, I1 and I2 having a molecular weight from 5000 to 30000, in particular from 20000 to 100000

Further preferred are polymers of formulae I, I1 and I2 wherein R¹ and R² are selected from C₁-C₂₀-alkyl that is optionally substituted with one or more fluorine atoms, C₁-C₂₀-alkenyl, C₁-C₂₀-alkynyl, C₁-C₂₀-alkoxy, C₁-C₂₀-thioether, C₁-C₂₀-silyl, C₁-C₂₀-ester, C₁-C₂₀-amino, C₁-C₂₀-fluoroalkyl, (CH₂CH₂O)ₘ, with m being from 1 to 20, and optionally substituted aryl or heteroaryl.

Further preferred are polymers of formula I1 and I2 wherein R³ and R⁴ are selected from H, halogen, Sn(R⁰)₃, CH₂Cl, COH, CH=CH_{2,} SiR⁰R⁰⁰ and optionally substituted aryl or heteroaryl.

Especially preferred aryl and heteroaryl groups are phenyl in which, in addition, one or more CH groups may be replaced by N, naphthalene, thiophene, thienothiophene, dithienothiophene, alkyl fluorene and oxazole, all of which can be unsubstituted, mono- or polysubstituted with L, wherein L is halogen or an alkyl, alkoxy, alkylcarbonyl or alkoxycarbonyl group with 1 to 12 C atoms, wherein one or more H atoms may be replaced by F or Cl.

If one of R¹ and R² is an alkyl or alkoxy radical, i.e. where the terminal CH₂ group is replaced by -O-, this may be straight-chain or branched. It is preferably straight-chain, has 2 to 8 carbon atoms and accordingly is preferably ethyl, propyl, butyl, pentyl, hexyl, heptyl, octyl, ethoxy, propoxy, butoxy, pentoxy, hexyloxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy, for example.

Oxaalkyl, i.e. where one CH₂ group is replaced by -O-, is preferably straight-chain 2-oxapropyl (=methoxymethyl), 2- (=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

Fluoroalkyl is preferably CᵢF₂ᵢ₊₁, wherein i is an integer from 1 to 15, in particular CF₃, C₂F₅, C₃F₇, C₄F₉, C₅F₁₁, C₆F₁₃, C₇F₁₅ or C₈F₁₇, very preferably C₆F₁₃.

Halogen is preferably F or Cl.

The polymers according to the invention are especially useful as charge transport semiconductors in that they have high carrier mobilities. Particularly preferred are polymers wherein the DTT group is substituted by one or more alkyl or fluoroalkyl groups. The introduction of fluoroalkyl and alkyl side chains into the DTT group improves their solubility and therefore their solution processibility. Furthermore, the presence of fluoroalkyl side chains also renders them effective as *n*-type semiconductors. The electron-withdrawing nature of the fluoroalkyl substituents will also lower the HOMO further and result in a more stable material, which is less susceptible to oxidation.

Furthermore, the polymers according to the present invention have good solution processibility. They are preferably prepared via soluble sulphonium precursor polymers (**9**) as depicted below in Scheme 1, in analogy to a route that was reported in the literature for polythiophenevinylene (PTV) [see reference 11, 12]. This route is particularly applicable to an all solution fabrication route in that after post heat treatment, the polymer will have significantly lower solubility and thus be inert to the subsequent solution processing route. 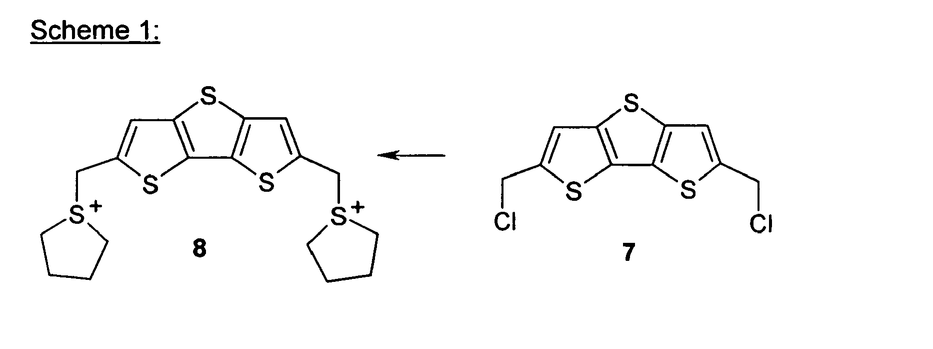 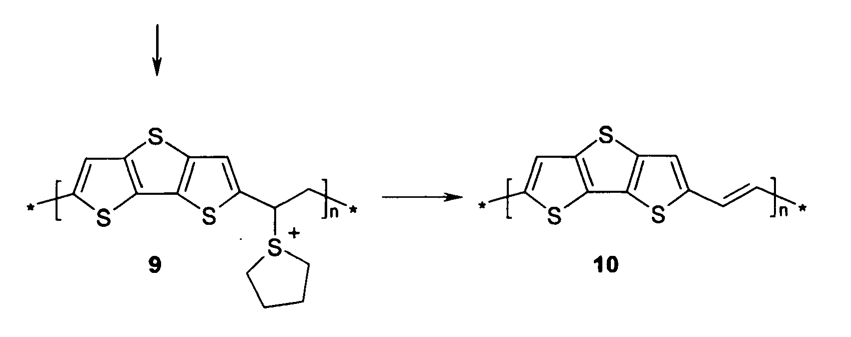 The sulphonium precursor polymer (**9**) has high solubility and good processibility, e.g. during the formation of thin film devices, and can be converted in situ, after film formation, into the fully conjugated conductive polymer (**10**) for example by heat treatment.

The sulphonium precursor polymers of the novel polymers are another aspect of the invention.

Especially preferred are precursor polymers having identical or different recurring units of formula la 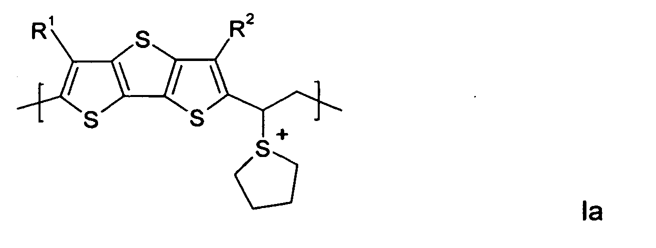 wherein R¹ and R² have independently of each other one of the meanings of formula I or the preferred meanings given above.

Especially preferred are precursor polymers of formula I1a 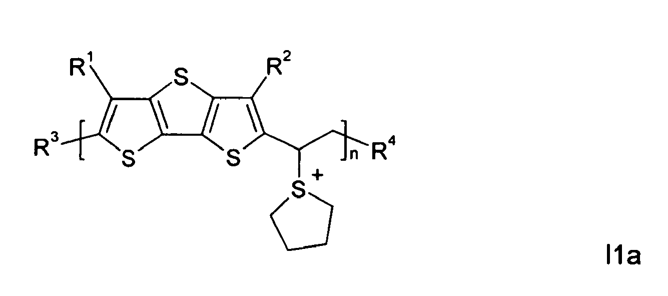 wherein R¹, R², R³, R⁴ and n are as defined in formula I1 and wherein the recurring units may be identical or different.

Another aspect of the invention relate to a method of forming a thin film, preferably with a thickness < 1 micron, of a conjugated polymer according to the present invention, by applying a sulphonium precursor polymer of the conjugated polymer to a substrate, preferably from solution by known methods like for example spin-coating or common roll to roll processing techniques such as reverse gravure, followed by conversion of the precursor polymer into the conjugated polymer, for example by heat treatment

The polymers of the present invention can be synthesized according to or in analogy to known methods. Some preferred methods are described below.

### Synthesis of unsubstituted poly (dithienothiophene vinylenes) (PDTTVs) (Scheme 2)

The bischloromethyl-DTT **(13)** is reacted with butanethiol and sodium hydroxide in the presence of a phase transfer catalyst in water to form the sulphide **(24).** The sulphide is oxidised to the sulphoxide **(25)** with hydrogen peroxide and TeO2 in methanol. Treatment of the monomer **(25)** with 1 equivalent of potassium *tert*-butoxide, followed by rapid quenching gives the sulphonium precursor polymer (**26**). This precursor polymer is soluble, for example during device formation can be deposited by spin coating onto the device. Heat treatment and elimination then leads to the required polymer (**16**).

This route also has the advantage of giving an insoluble polymer that will not be effected by the coating of subsequent layers.

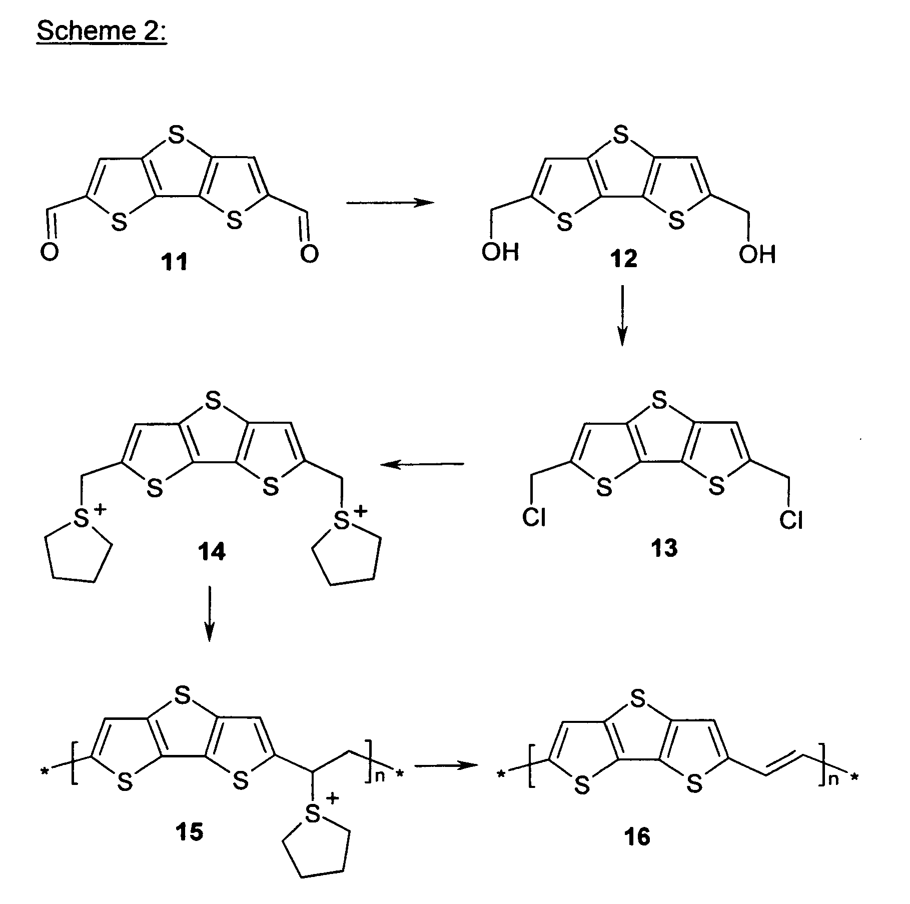

### Synthesis of unsubstituted PDTTVs (Scheme 3)

The bischloromethyl-DTT **(13)** is reacted with butanethiol and sodium hydroxide in the presence of a phase transfer catalyst in water to form the sulphide **(24).** The sulphide is oxidised to the sulphoxide **(24)** with hydrogen peroxide and TeO₂ in methanol. Treatment of the monomer **(24)** with 1 equivalent of potassium *tert*-butoxide, followed by rapid quenching gives the sulphonium precursor polymer (**25**).

This precursor polymer is soluble, for example during device formation can be deposited by spin coating onto the device. Heat treatment and elimination then leads to the required polymer (**16**). This route also has the advantage of giving an insoluble polymer that will not be effected by the coating of subsequent layers.

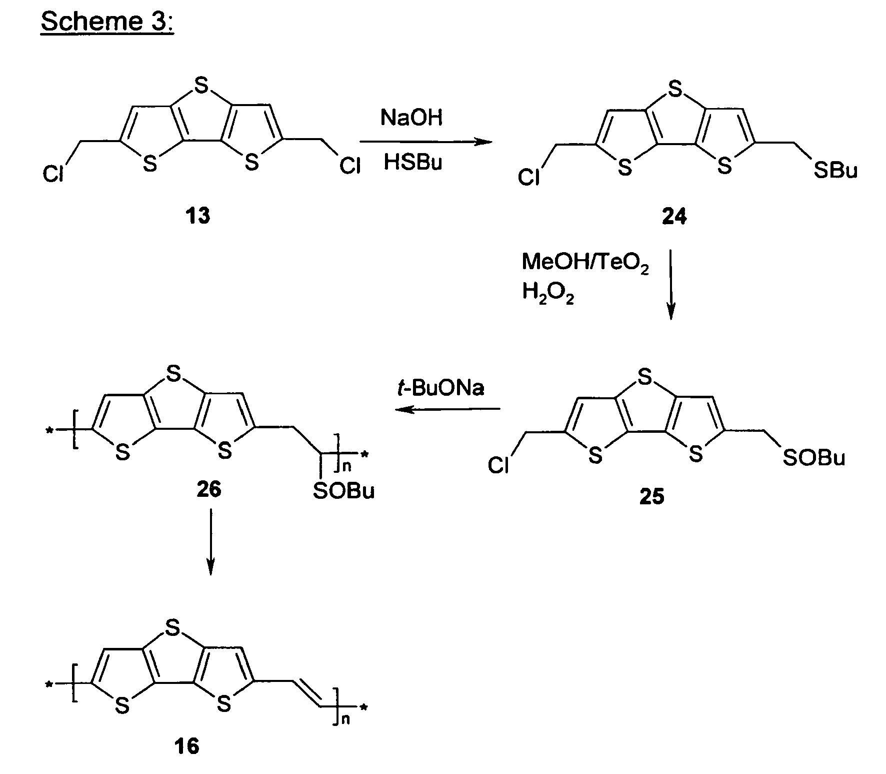

### Synthesis of substituted PDTTVs

Materials containing DDT that is substituted, for example with alkyl groups such as dihexyl DTT, have greater solubility than those containing unsubstituted DTT. This therefore increases the possible routes to vinylidene polymers [see reference 12].

### McMurry route (Scheme 4):

The Diformyl DTT derivative (**17**) can be reacted directly with titanium tetrachloride and zinc to yield directly the required polymer **(19).**

### Gilch and Wessling route (Scheme 4):

The Diformyl DTT derivative **(17)** can be reduced to the diol and then readily converted to the bischloromethyl-DTT (**18**). The bischloromethyl derivative can then be directly polymerised under Gilch conditions in the presence of potassium *tert*-butoxide to give a processable polymer (**19**). Alternatively reaction with tetrahydrothiophene yields the sulphonium monomer **(20).** Treatment of the monomer **(20)** with 1 equivalent of potassium *tert*-butoxide, followed by rapid quenching results in the sulphonium precursor **(21).** This is water-soluble and, for example during device formation can be deposited by spin coating onto the device. Heat treatment and elimination then leads to the required polymer (**19**). This route also has the advantage of giving an insoluble polymer that will not be affected by the coating of subsequent layers.

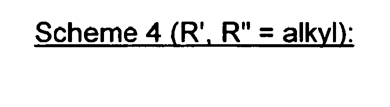

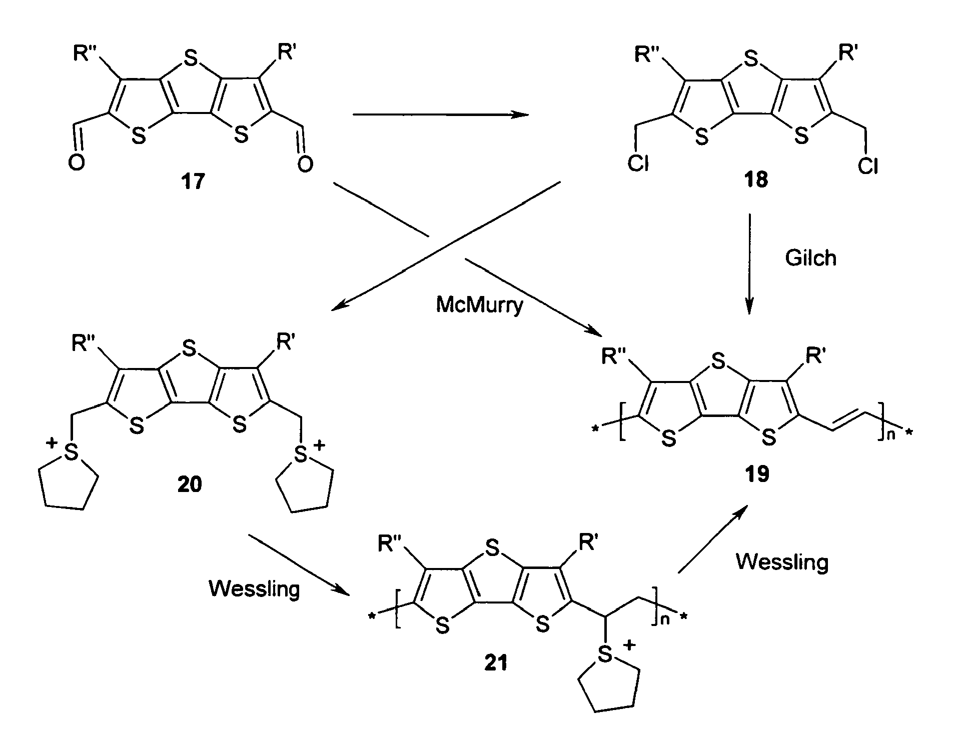

### Stille routes (Scheme 5)

The PDTTVs can also be made by cross coupling reaction. The dibromo DTT (**22**) can be coupled with bis(tributyl stannyl) ethene (**23**) under Stille conditions to yield the required polymer (**19**).

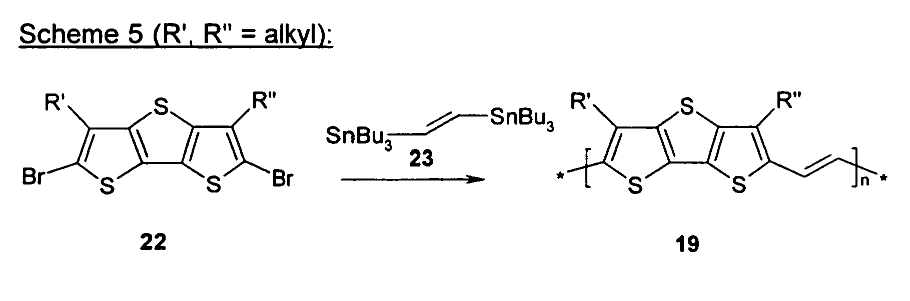

### Alternative precursor route (Scheme 6)

The bischloromethyl-DTT (**18**) is reacted with butanethiol and sodium hydroxide in the presence of a phase transfer catalyst in water to form the sulphide **(27).** The sulphide is oxidised to the sulphoxide **(28)** with hydrogen peroxide and TeO₂ in methanol. Treatment of the monomer **(28)** with 1 equivalent of potassium *tert*-butoxide, followed by rapid quenching gives the sulphonium precursor polymer (**29**). This precursor polymer is soluble, for example during device formation can be deposited by spin coating onto the device. Heat treatment and elimination then leads to the required polymer (**19**). This route also has the advantage of giving an insoluble polymer that will not be effected by the coating of subsequent layers.

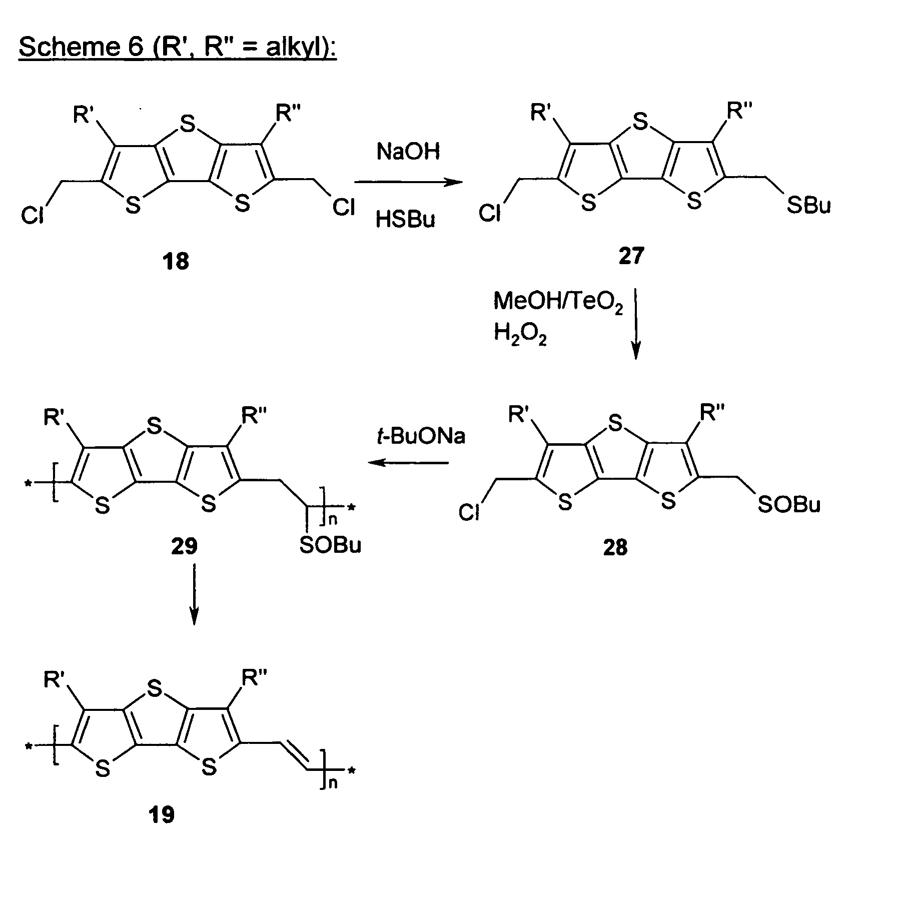

### Synthesis of poly(dithienothiophene ethinylenes) (Scheme 7)

DTT polymers containing the ethyne moiety can be made as shown below in scheme 7. Utilising the Stille methodology the dibromo DTT (**22**) can be coupled with bis(tributyl stannyl) ethyne (**25**) to yield the required polymer (**41**).

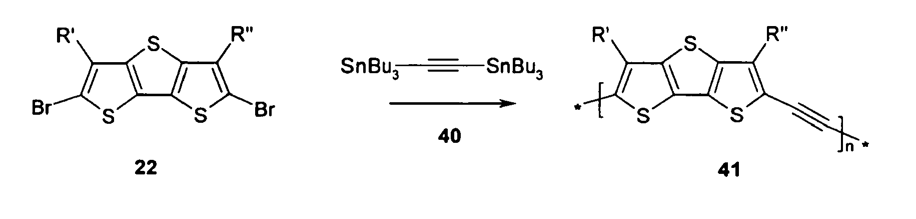

A further aspect of the invention relates to both the oxidised and reduced form of the polymers according to this invention. Either loss or gain of electrons results in formation of a highly delocalised ionic form, which is of high conductivity. This can occur on exposure to common dopants. Suitable dopants and methods of doping are known to those skilled in the art, e.g. from EP 0 528 662, US 5,198,153 or WO 96/21659.

The doping process typically implies treatment of the semiconductor material with an oxidating or reducing agent in a redox reaction to form delocalised ionic centres in the material, with the corresponding counterions derived from the applied dopants. Suitable doping methods comprise for example exposure to a doping vapor in the atmospheric pressure or at a reduced pressure, electrochemical doping in a solution containing a dopant, bringing a dopant into contact with the semiconductor material to be thermally diffused, and ion-implantantion of the dopant into the semiconductor material.

When electrons are used as carriers, suitable dopants are for example halogens (e.g. I₂, Cl₂, Br₂, ICI, ICl₃, IBr and IF), Lewis acids (e.g. PF₅, AsF₅, SbF₅, BF₃, BCl₃, SbCl₅, BBr₃ and SO₃), protonic acids, organic acids, or amino acids (e.g. HF, HCI, HNO₃, H₂SO₄, HClO₄, FSO₃H and ClSO₃H), transition metal compounds (e.g. FeCl₃, FeOCl, Fe(ClO₄)₃, Fe(4-CH₃C₆H₄SO₃)₃, TiCl₄, ZrCl₄, HfCl₄, NbF₅, NbCl₅, TaCl₅, MoF₅, MoCl₅, WF₅, WCl₆, UF₆ and LnCl₃ (wherein Ln is a lanthanoid), anions (e.g. Cl⁻, Br⁻, I⁻, I₃⁻, HSO₄⁻, SO₄²⁻, NO₃⁻, ClO₄⁻, BF₄⁻, PF₆⁻, AsF₆⁻, SbF₆⁻, FeCl₄⁻, Fe(CN)₆³⁻, and anions of various sulfonic acids, such as aryl-SO₃⁻). When holes are used as carriers, examples of dopants are cations (e.g. H⁺, Li⁺, Na⁺, K⁺, Rb⁺ and Cs⁺), alkali metals (e.g., Li, Na, K, Rb, and Cs), alkaline-earth metals (e.g., Ca, Sr, and Br), O₂, XeOF₄, (NO₂⁺) (SbF₆⁻), (NO₂⁺) (SbCl₆⁻), (NO₂⁺) (BF₄⁻), AgClO₄, H₂lrCl₆, La(NO₃)₃ 6H₂O, FSO₂OOSO₂F, Eu, acetylcholine, R₄N⁺, (R is an alkyl group), R₄P⁺ (R is an alkyl group), R₆As⁺ (R is an alkyl group), and R₃S⁺ (R is an alkyl group).

The conducting form of the compounds and materials of the present invention can be used as an organic "metal" in applications, for example, but not limited to, charge injection layers and ITO planarising layers in organic light emitting diode applications, films for flat panel displays and touch screens, antistatic films, printed conductive substrates, patterns ot tracts in electronic applications such as printed circuit boards and condensers.

The polymers according to the present invention are useful as optical, electronic and semiconductor materials, in particular as charge transport materials in field effect transistors (FETs) e.g. as components of integrated circuitry, ID tags or TFT applications. Alternatively, they may be used in organic light emitting diodes (OLEDs) in electroluminescent display applications or as backlight of e.g. liquid crystal displays, as photovoltaics or sensor materials, for electrophotographic recording, and for other semiconductor applications.

The polymers according to the invention show advantageous solubility properties which allow production processes using solutions of these compounds. Thus films, including layers and coatings, may be generated by low cost fabrication techniques e.g. roll to roll solution coating. Suitable solvents or solvent mixtures comprise alkanes and/ or aromatics, especially their fluorinated derivatives.

The polymers according to the present invention are useful as optical, electronic and semiconductor materials, in particular as charge transport materials in field effect transistors (FETs), as photovoltaics or sensor materials, for electrophotographic recording, and for other semiconductor applications. Such FETs, where an organic semiconductive material is arranged as a film between a gate-dielectric and a drain and a source electrode, are generally known e.g. from US 5,892,244, WO 00/79617, US 5,998,804, and from the references cited in the background and prior art chapter and listed below. Due to the advantages associated with this material, like low cost fabrication using the solubility properties of the materials according to the invention and thus the processibility of large surfaces, preferred applications of these FETs are such as integrated circuitry, TFT-displays and security applications.

In security applications, field effect transistors and other devices with semiconductive materials, like transistors or diodes, may be used for ID tags or security markings to authenticate and prevent counterfeiting of documents of value like banknotes, credit cards or ID cards, national ID documents, licenses or any product with money value, like stamps, tickets, shares, cheques etc..

Alternatively, the polymers according to the invention may be used in organic light emitting devices or diodes (OLEDs), e.g. in display applications or as backlight of e.g. liquid crystal displays. Common OLEDs are realized using multilayer structures. An emission layer is generally sandwiched between one or more electron-transport and/ or hole-transport layers. By applying an electric voltage electrons and holes as charge carriers move towards the emission layer where their recombination leads to the excitation and hence luminescence of the lumophor units contained in the emission layer. The inventive polymers may be employed in one or more of the charge transport layers and/ or in the emission layer, corresponding to their electrical and/ or optical properties. Furthermore their use within the emission layer is especially advantageous, if the polymers according to the invention show electroluminescent properties themselves or comprise electroluminescent groups or compounds. The selection, characterization as well as the processing of suitable monomeric, oligomeric and polymeric compounds or materials for the use in OLEDs is generally known by a person skilled in the art, see e. g. Meerholz, Synthetic Materials, 111-112, 2000, 31-34, Alcala, J. Appl. Phys., 88, 2000, 7124-7128 and the literature cited therein.

According to another use, the inventive polymers, especially those which show photoluminescent properties, may be employed as materials of light sources, e.g. of display devices such as described in EP 0 889 350 A1 or by C. Weder et al., Science, 279, 1998, 835-837.

### References:

1. H. E. Katz, Z. Bao and S. L. Gilat, Acc. Chem. Res., 2001, 34, 5, 359.
2. Sirringhaus, R. H. Friend, X. C. Li, S. C. Moratti, A. B. Holmes and N. Feeder, Appl. Phys. Lett., 1997, 71, 26, 3871.
3. X. C. Li, H. Sirringhaus, F. Garnier, A. B. Holmes, S. C. Moratti, N. Feeder, W. Clegg, S. J. Teat and R. H. Friend, J. Am. Chem. Soc., 1998, 120, 2206
4. J. J. Morrison, M. M. Murray, X. C.Li, A. B. Holmes, S. C. Moratti, R. H. Friend and H. Sirringhaus, Synth. Met., 1999, 102, 987.
5. Arbizzani, C.; Catellani, M.; Mastragostino, M.; Cerroni, M. G. Journal of Electroanalytical Chemistry 1997, 423, 23-28.
6. Quattrochi, C.; Lazzaroni, R.; Brédas, J. I.; Zamboni, R.; C, T. Macromolecules 1993, 26, 1260-1264.
7. Campos, M.; Casalbore-Miceli, G.; Camaioni, N. Journal of Physics D: Applied Physics 1995, 28, 2123-2127.
8. Cervani, R.; Holmes, A. B.; Moratti, S. C.; Köhler, A.; Friend, R. H. Synthetic Metals 1996, 76, 169-171.
9. Koßmehl, G.; Belmling, P.; Manecke, G. Makromolekulare Chemie 1982, 183, 2771-2786.
10. WO 99/12989.
11. Fuchigami, H.; Tsumura, A.; H, K. Applied Physics Letters 1993, 63, 1372-1374.
12. Kraft, A.; Grimsdale, A. C.; Holmes, A. B. Angewante Chemie International version 1998, 37, 402-428

## Claims

1. Conjugated polymer having identical or different recurring units selected of formula I 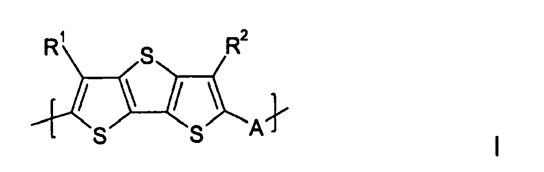 wherein
R¹ and R² are independently of each other H, halogen or straight chain, branched or cyclic alkyl with 1 to 20 C-atoms, which may be unsubstituted, mono- or poly-substituted by F, Cl, Br, I or CN, it being also possible for one or more non-adjacent CH₂ groups to be replaced, in each case independently from one another, by -O-, -S-, -NH-, -NR⁰-, -SiR⁰R⁰⁰-,-CO-, -COO-, -OCO-, -OCO-O-, -S-CO-, -CO-S-,-CH=CH- or -C≡C- in such a manner that O and/or S atoms are not linked directly to one another, or optionally substituted aryl or heteroaryl,
R⁰ and R⁰⁰ are independently of each other H or alkyl with 1 to 12 C-atoms,
A is -CX¹=CX²- or -C≡C-, and
X¹ and X² are independently of each other H, F, Cl or CN.

2. Conjugated polymer according to claim 1, selected of formula I1 and I2 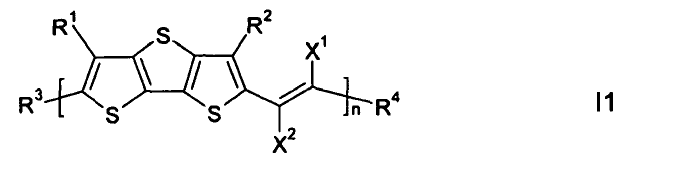 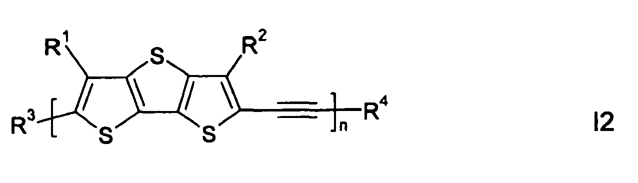 wherein
R¹ and R² have independently of each other one of the meanings given in claim 1,
R³ and R⁴ are independently of each other H, halogen, Sn(R⁰)3 or straight chain, branched or cyclic alkyl with 1 to 20 C-atoms, which may be unsubstituted, mono- or poly-substituted by F, Cl, Br, I, -CN and/ or -OH, it being also possible for one or more non-adjacent CH₂ groups to be replaced, in each case independently from one another, by -O-, -S-, -NH-,-NR⁰-, -SiR⁰R⁰⁰-, -CO-, -COO-, -OCO-, -OCO-O-,-S-CO-, -CO-S-, -CH=CH- or -CC- in such a manner that O and/or S atoms are not linked directly to one another, or optionally substituted aryl or heteroaryl,
n is an integer from 2 to 5000,
and wherein the recurring units may be identical or different.

3. A sulphonium precursor polymer having identical or different recurring units selected of formula la 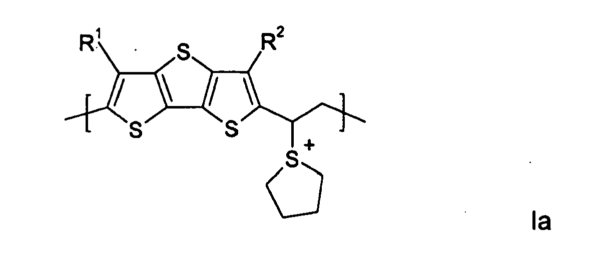 wherein R¹ and R² have independently of each other one of the meanings given in claim 1.

4. A precursor polymer of claim 3, selected of formula I1a 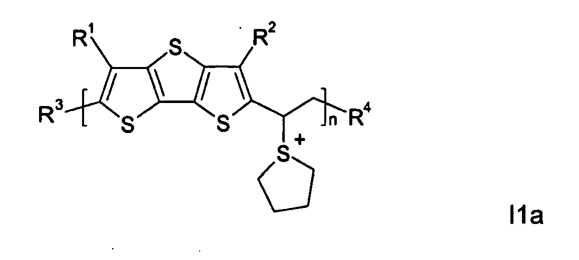 wherein R¹, R², R³, R⁴ and n have the meanings given in claim 1 and 2, and wherein the recurring units may be identical or different.

5. Method of forming a thin film of a conjugated polymer according to claim 1, wherein A is -CH=CH-, by applying a sulphonium precursor polymer according to claim 3 to a substrate from solution, followed by conversion of the sulphonium precursor polymer into the conjugated polymer by heat treatment.

6. Method according to claim 5, wherein the conjugated polymer is of formula I1 as defined in claim 2, wherein X¹ and X² are H, and the sulphonium precursor polymer is of formula I1a as defined in claim 4.

7. Polymer or method according to one or more of claims 1 to 6, wherein R¹ and R² are selected from C₁-C₂₀-alkyl that is optionally substituted with one or more fluorine atoms, C₁-C₂₀-alkenyl, C₁-C₂₀-alkynyl, C₁-C₂₀-alkoxy, C₁-C₂₀-thioether, C₁-C₂₀-silyl, C₁-C₂₀-ester, C₁-C₂₀-amino, C₁-C₂₀-fluoroalkyl, (CH₂CH₂O)ₘ, with m being from 1 to 20, and optionally substituted aryl or heteroaryl.

8. Polymer or method according to one or more of claims 1 to 7, wherein R³ and R⁴ are selected from H, halogen, Sn(R⁰)₃ , CH₂Cl, COH, CH=CH₂, SiR⁰R⁰⁰ and optionally substituted aryl or heteroaryl.

9. Polymer or method according one or more of claims 1 to 8, wherein the degree of polymerisation or n is from 10 to 5000.

10. Use of a polymer according to one or more of claims 1 to 9 as semiconductor or charge transport material in optical, electrooptical or electronic devices, in components of integrated circuitry, in field effect transistors for example as thin film transistors in flat panel display applications or for RFID tags, and in semiconducting components for organic light emitting diode (OLED) applications, electroluminescent display devices, backlights, photovoltaic or sensor devices, and for electrophotographic applications.

11. Field effect transistor (FET), OLED, electroluminescent device, RFID tag, backlight, photovoltaic or sensor device or electrophotographic recording device comprising one or more polymers according to one or more of claims 1 to 9.

12. Security marking or device comprising one or more polymers according to one or more of claims 1 to 9 or a FET or RFID tag according to claim 11.

13. Polymer according to one or more of claims 1 to 9 which is oxidatively or reductively doped.

14. Charge injection layer, planarising layer, antistatic film or conducting substrate or pattern for electronic applications or flat panel displays, comprising one or more polymers according to claim 13.

## Patentansprüche

1. Konjugiertes Polymer mit gleichen oder verschiedenen wiederkehrenden Einheiten ausgewählt aus der Formel I 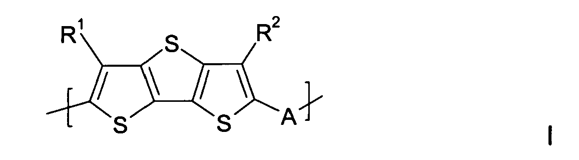 worin
R¹ und R² unabhängig voneinander H, Halogen oder geradkettiges, verzweigtes oder cyclisches Alkyl mit 1 bis 20 C-Atomen, welches unsubstituiert oder ein- oder mehrfach durch F, Cl, Br, I oder CN substituiert sein kann, wobei auch eine oder mehrere nicht benachbarte CH₂-Gruppen jeweils unabhängig voneinander so durch -O-, -S-, -NH-, -NR⁰-, -SiR⁰R⁰⁰-, -CO-, -COO-, -OCO-, -OCO-O-, -S-CO-, -CO-S-, -CH=CH- oder -C≡C- ersetzt sein können, dass O-und/oder S-Atome nicht direkt miteinander verknüpft sind, oder gegebenenfalls substituiertes Aryl oder Heteroaryl bedeuten,
R⁰ und R⁰⁰ unabhängig voneinander H oder Alkyl mit 1 bis 12 C-Atomen bedeuten,
A -CX¹=CX²- oder -C≡C- bedeutet und
X¹ und X² unabhängig voneinander H, F, Cl oder CN bedeuten.

2. Konjugiertes Polymer nach Anspruch 1, ausgewählt aus den Formeln I1 und I2 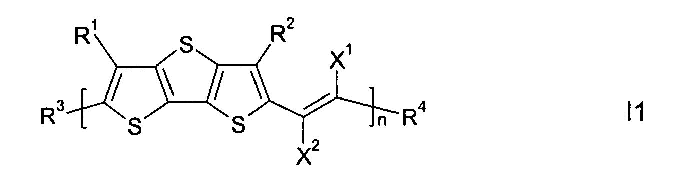 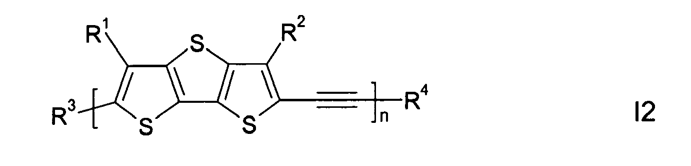 worin
R¹ und R² unabhängig voneinander eine der in Anspruch 1 angegebenen Bedeutungen besitzen,
R³ und R⁴ unabhängig voneinander H, Halogen, Sn(R⁰)₃ oder geradkettiges, verzweigtes oder cyclisches Alkyl mit 1 bis 20 C-Atomen, welches unsubstituiert oder ein-oder mehrfach durch F, Cl, Br, I, -CN und/oder -OH substituiert sein kann, wobei auch eine oder mehrere nicht benachbarte CH₂-Gruppen jeweils unabhängig voneinander so durch -O-, -S-, -NH-, -NR⁰-, -SiR⁰R⁰⁰-, -CO-, -COO-, -OCO-, -OCO-O-, -S-CO-, -CO-S-, -CH=CH- oder -C≡C- ersetzt sein können, dass O- und/oder S-Atome nicht direkt miteinander verknüpft sind, oder gegebenenfalls substituiertes Aryl oder Heteroaryl bedeuten,
n eine ganze Zahl von 2 bis 5000 bedeutet,
und worin die wiederkehrenden Einheiten gleich oder verschieden sein können.

3. Sulfonium-Vorläuferpolymer mit gleichen oder verschiedenen wiederkehrenden Einheiten ausgewählt aus der Formel Ia 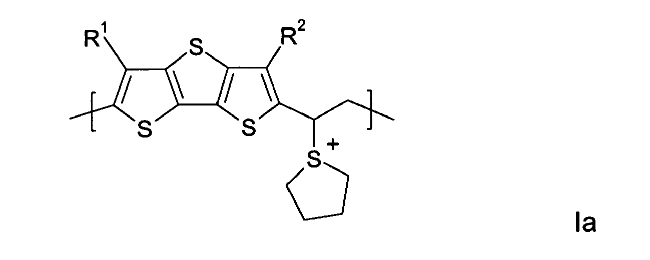 worin R¹ und R² unabhängig voneinander eine der in Anspruch 1 angegebenen Bedeutungen besitzen.

4. Vorläuferpolymer nach Anspruch 3, ausgewählt aus der Formel I1a 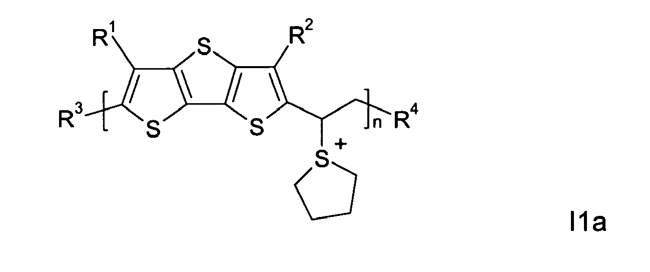 worin R¹, R², R³, R⁴ und n die in Anspruch 1 und 2 angegebenen Bedeutungen besitzen und worin die wiederkehrenden Einheiten gleich oder verschieden sein können.

5. Verfahren zur Bildung eines dünnen Films eines konjugierten Polymers nach Anspruch 1, worin A -CH=CH- bedeutet, durch Aufbringen eines Sulfonium-Vorläuferpolymers nach Anspruch 3 aus der Lösung auf ein Substrat und nachfolgende Umwandlung des Sulfonium-Vorläuferpolymers in das konjugierte Polymer durch Wärmeeinwirkung.

6. Verfahren nach Anspruch 5, worin das konjugierte Polymer die Formel I1 wie in Anspruch 2 definiert aufweist, worin X¹ und X² H bedeuten, und das Sulphonium-Vorläuferpolymer die Formel I1a wie in Anspruch 4 definiert aufweist.

7. Polymer oder Verfahren nach einem oder mehreren der Ansprüche 1 bis 6, worin R¹ und R² aus C₁-C₂₀-Alkyl, das gegebenenfalls mit einem oder mehreren Fluoratomen substituiert ist, C₁-C₂₀-Alkenyl, C₁-C₂₀-Alkinyl, C₁-C₂₀-Alkoxy, C₁-C₂₀-Thioether, C₁-C₂₀-Silyl, C₁-C₂₀-F-ster, C₁-C₂₀-Amino, C₁-C₂₀-Fluoralkyl, (CH₂CH₂O)ₘ, wobei m für 1 bis 20 steht, und gegebenenfalls substituiertem Aryl oder Heteroaryl ausgewählt sind.

8. Polymer oder Verfahren nach einem oder mehreren der Ansprüche 1 bis 7, worin R³ und R⁴ aus H, Halogen, Sn(R⁰)₃, CH₂Cl, COH, CH=CH₂, SiR⁰R⁰⁰ und gegebenenfalls substituiertem Aryl oder Heteroaryl ausgewählt sind.

9. Polymer oder Verfahren nach einem oder mehreren der Ansprüche 1 bis 8, worin der Polymerisationsgrad oder n 10 bis 5000 ist.

10. Verwendung eines Polymers nach einem oder mehreren der Ansprüche 1 bis 9 als Halbleiter oder Ladungstransportmaterial in optischen, elektrooptischen oder elektronischen Vorrichtungen, in Komponenten integrierter Schaltungen, in Feldeffekttransistoren, beispielsweise als Dünnfilmtransistoren in Flachbildschirmanwendungen oder für RFID-Tags (RFID - Radio Frequency Identification), und in Halbleiterkomponenten für Anwendungen mit organischen Leuchtdioden (OLED - organic light emitting diode), Elektrolumineszenzanzeigevorrichtungen, Hintergrundbeleuchtungen, Photovoltaik- oder Sensorvorrichtungen, und für elektrophotographische Anwendungen.

11. Feldeffekttransistor (FET), OLED, Elektrolumineszenzvorrichtung, RFID-Tag, Hintergrundbeleuchtung, Photovoltaik- oder Sensorvorrichtung oder elektrophotographische Aufzeichnungsvorrichtung enthaltend ein oder mehrere Polymere nach einem oder mehreren der Ansprüche 1 bis 9.

12. Sicherheitsmarkierung oder -vorrichtung enthaltend ein oder mehrere Polymere nach einem oder mehreren der Ansprüche 1 bis 9 oder einen FET oder ein RFID-Tag nach Anspruch 11.

13. Polymer nach einem oder mehreren der Ansprüche 1 bis 9, das oxidativ oder reduktiv dotiert ist.

14. Ladungsinjektionsschicht, Planarisierungsschicht, Antistatikfolie oder leitendes Substrat oder leitende Struktur für Elektronikanwendungen oder Flachbildschirme, enthaltend ein oder mehrere Polymere nach Anspruch 13.

## Revendications

1. Polymère conjugué ayant des unités récurrentes identiques ou différentes choisies à partir de la formule I 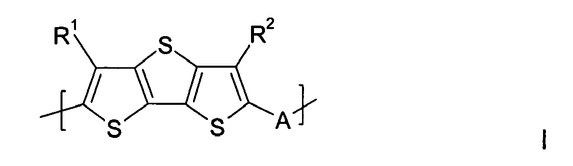 dans laquelle
R¹ et R² sont indépendamment l'un de l'autre H, halogène ou alkyle en chaîne droite, ramifié ou cyclique avec 1 à 20 atomes de C, qui peut être non substitué, mono- ou poly-substitué par F, CI, Br, I ou CN, étant également possible qu'un ou plusieurs groupes CH₂ non adjacents soient remplacés, dans chaque cas indépendamment l'un de l'autre, par -O-, -S-, -NH-, -NR⁰-, -SiR⁰R⁰⁰-, -CO-, -COO-, -OCO-, -OCO-O-, -S-CO-, -CO-S-, -CH=CH- ou -C≡C- de telle sorte que des atomes de O et/ou S ne soient pas liés directement l'un à l'autre, ou aryle ou hétéroaryle optionnellement substitué,
R⁰ et R⁰⁰ sont indépendamment l'un de l'autre H ou alkyle avec 1 à 12 atomes de C,
A est -CX¹=CX²- ou -C≡C-, et
X¹ et X² sont indépendamment l'un de l'autre H, F, Cl ou CN.

2. Polymère conjugué selon la revendication 1, choisi à partir de la formule I1 et I2 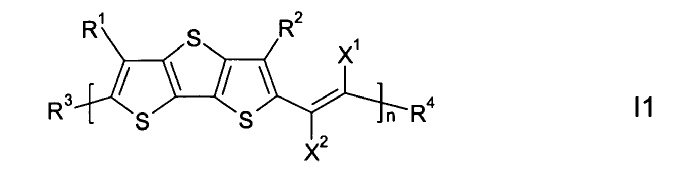 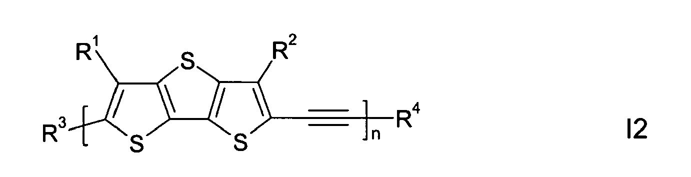 dans lesquelles
R¹ et R² ont indépendamment l'un de l'autre une des significations données dans la revendication I,
R³ et R⁴ sont indépendamment l'un de l'autre H, halogène, Sn(R⁰)₃ ou alkyle en chaîne droite, ramifié ou cyclique avec 1 à 20 atomes de C, qui peut être non substitué, mono- ou poly-substitué par F, CI, Br, I, -CN et/ou -OH, étant également possible qu'un ou plusieurs groupes CH₂ non adjacents soient remplacés, dans chaque cas indépendamment l'un de l'autre, par -O-, -S-, -NH-, -NR⁰-, -SiR⁰R⁰⁰-, -CO-, -COO-, -OCO-, -OCO-O-, -S-CO-, -CO-S-, -CH=CH- ou -C≡C- de telle sorte que des atomes de O et/ou S ne soient pas liés directement l'un à l'autre, ou aryle ou hétéroaryle optionnellement substitué,
n est un entier de 2 à 5000,
et dans lesquelles les unités récurrentes peuvent être identiques ou différentes.

3. Polymère précurseur de sulphonium ayant des unités récurrentes identiques ou différentes choisies à partir de la formule la 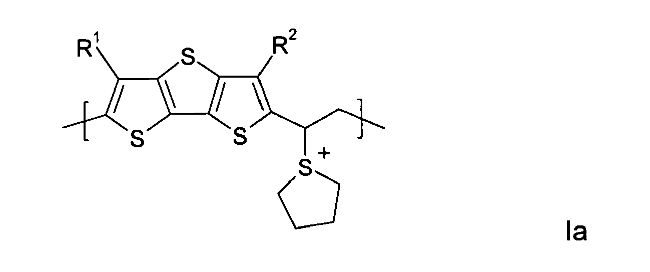 dans laquelle R¹ et R² ont indépendamment l'un de l'autre les significations données dans la revendication 1.

4. Polymère précurseur selon la revendication 3, choisi à partir de la formule I1a 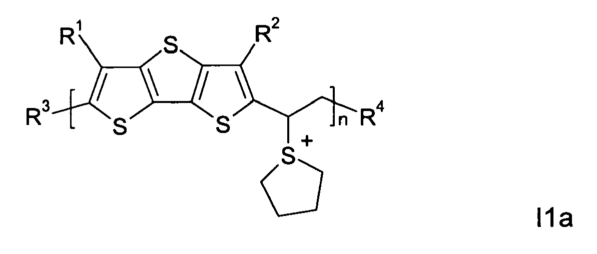 dans laquelle R¹, R², R³, R⁴ et n ont les significations données dans la revendication 1 et 2, et dans laquelle les unités récurrentes peuvent être identiques ou différentes.

5. Procédé de formation d'un film mince d'un polymère conjugué selon la revendication 1, dans lequel A est -CH=CH-, par l'application d'un polymère précurseur de sulphonium selon la revendication 3 à un substrat à partir d'une solution, suivi par la conversion du polymère précurseur de sulphonium selon le polymère conjugué par traitement thermique.

6. Procédé selon la revendication 5, dans lequel le polymère conjugué est de la formule I1 comme définie dans la revendication 2, dans lequel X¹ et X² sont H, et le polymère précurseur de sulphonium est de la formule I1e comme définie dans la revendication 4.

7. Polymère ou procédé selon une ou plusieurs des revendications 1 à 6, dans lequel R¹ et R² sont choisis parmi C₁-C₂₀-alkyle qui est optionnellement substitué avec un ou plusieurs atomes de fluor, C₁-C₂₀-alkényle, C₁-C₂₀-alkynyle, C₁-C₂₀-alcoxy, C₁-C₂₀-thioéther, C₁-C₂₀-silyle, C₁-C₂₀-ester, C₁-C₂₀-amino, C₁-C₂₀-fluoroalkyle, (CH₂CH₂O)ₘ, m étant de 1 à 20, et aryle ou hétéroaryle optionnellement substitué.

8. Polymère ou procédé selon une ou plusieurs des revendications 1 à 7, dans lequel R³ and R⁴ sont choisis parmi H, halogène, Sn(R⁰)₃, CH₂Cl, COH, CH=CH₂, SiR⁰R⁰⁰ et aryle ou hétéroaryle optionnellement substitué.

9. Polymère ou procédé selon une ou plusieurs des revendications 1 à 8, dans lequel le degré de polymérisation ou n est de 10 à 5000.

10. Utilisation d'un polymère selon une ou plusieurs des revendications 1 à 9 comme semiconducteur ou matériau de transport de charges dans des dispositifs optiques, électrooptiques ou électroniques, dans des composants de circuits intégrés, dans des transistors à effet de champ par exemple comme transistors à film mince dans des applications d'affichage à écran plat ou pour des étiquettes RFID, et dans des composants de semiconduction pour des applications de diodes émettrices de lumière organiques (OLED), des dispositifs d'affichage électroluminescent, des éclairages arrières, des dispositifs photovoltaïques ou de capteurs, et pour des applications électrophotographiques.

11. Transistor à effet de champ (FET), OLED, dispositif électroluminescent, étiquette RFID, éclairage arrière, dispositif photovoltaïque ou de capteur ou dispositif d'enregistrement électrophotographique comprenant un ou plusieurs polymères selon une ou plusieurs des revendications 1 à 9.

12. Marquage ou dispositif de sécurité comprenant un ou plusieurs polymères selon une ou plusieurs des revendications 1 à 9 ou un FET ou une étiquette RFID selon la revendication 11.

13. Polymère selon une ou plusieurs des revendications 1 à 9 qui est dopé de façon oxydante ou réductrice.

14. Couche d'injection de charges, couche de planarisation, film antistatique ou substrat ou motif de conduction pour des applications électroniques ou des affichages à écran plat, comprenant un ou plusieurs polymères selon la revendication 13.
